# EUROPEAN PATENT APPLICATION

(11) **EP 2 363 426 A1**
(43) Date of publication of application: **07.09.2011**
(21) Application number: 10154860.0
(22) Date of filing: 26.02.2010
(51) Int. Cl.: C08G 81/00, A61F 2/14, A61F 2/16, C08G 65/00, C08G 77/00, C08L 71/02, C08L 83/00, G02C 7/04

(54) **Organic compositions for repeatedly adjustable optical elements and such elements.**

(71) Applicant: Université de Liège, 4031 Angleur (BE)
(72) Inventor: Jerome, Christine, 4000, LIEGE (BE)

(57) **Abstract**

The invention relates to an organic transparent liquid composition comprising preformed polymeric chains having some photoactive groups per chain that can reversibly couple, being capable in this way to form a transparent solid polymer network with a shape that can be repeatedly and reversibly adjusted by at least local irradiation of the network with electromagnetic waves of different wave lengths whereby the liquid composition is cross-linked at at least one wave length L1 on the one hand and is liquid again by cleaving the cross-link nodes by irradiating the material at at least one other wavelength L2 on the other hand in order to repeatedly adjust the optical properties of said composition in its photo-cross linked state.

The composition is applicable as a starting material for intraocular lenses and for other lenses and optical elements.

## Description

### FIELD OF THE INVENTION

The invention relates to organic compositions for repeatedly adjustable optical elements and such elements as well as their applications, in particular for intraocular lenses.

### BACKGROUND OF THE INVENTION

Intraocular lenses (IOLs) are intended for the replacement of the opacified human lens during a cataract surgery. The usual treatment of cataract is to surgically remove the cloudy lens by an ultrasonic method (phaco-emulsification) and to implant an artificial synthetic IOL.

One of the problems with conventional cataract surgeries relates to the fact that, prior to the surgery it is virtually impossible to select the lens to be implanted in a way as to avoid further post-invasive vision corrections. Indeed, the IOL can be removed only by another surgical intervention and associated risks. Also accommodation, i.e. the ability to view objects at both near and far distances is often altered with ageing. This is another problem with the IOL's of the state of the art that do not allow a repeated adjustment.

Multi-focal IOLs have also been developed, working much like bifocal or trifocal eyeglasses. Often patients with multi-focal IOLs do not get postoperatively used with the type of multi-focal lens because of optical secondary phenomenon (halos etc) and/or insufficient brain plasticity. Again in that case, the only way to solve this inconvenience is to proceed to lens exchange by another surgical intervention with its associated risks.

IOLs which can be post-operatively adjusted in a very limited way by photo-chemical means have been proposed in US 2007/0035689. A semi-finished lens is implanted by the standard procedure and further shaped in situ by laser assisted photo irradiation to define i.a. the refractive power. Such lens material can consist of a cross-linked PDMS network swollen by polymer precursors (monomers or macro-monomers) that later on, once introduced in the eye, can only irreversibly be further polymerised. However, this material suffers from some important drawbacks.

Firstly, the said polymer precursors are based on vinyl or acrylic groups that are quite reactive towards side reactions. This may induce toxicity to these products before final polymerisation in addition they are quite sensitive and can be altered by sunlight exposure of the patient in an uncontrolled manner. Therefore, the patient must wear dark or black sun spectacles for some time after surgery to avoid uncontrolled polymerisation, i.e. the time required for mechanical stabilisation of the IOL in the eye.

Secondly, a small organic molecule playing the role of photosensitizer has to be added to the lens formulation which is required for the final cross-linking process. However it is not covalently linked to the lens material. It can thus diffuse and be toxic for the eye. Finally the photo-polymerisation is irreversible. This means that the optical properties of the lens can be adjusted only once after implantation.

There is thus a need for a material that would avoid or at least encounter these drawbacks, not only for IOLs but likewise for other optical elements such as e.g. prisms and lenses. In general it is an object of the invention to enable a repeated adjustment, also in a reversible manner of e.g. the refractive index or dioptric power by a proper irradiation treatment of an organic optical element.

It is also known for IOLs to implant them through a very small incision in the empty capsular bag after removal from that bag of the opacified human lens during a cataract surgery. Actually, instead of implanting a rigid or semi-rigid lens (through a non desired larger incision in the capsular bag) a liquid composition is injected through the small incision and photo-polymerised in situ to a flexible transparent lens body. This body then properly fills up the capsular bag. However, here again the known liquid compositions do not allow a repeatedly adjustable and reversible change of e.g. the refractive index of the in situ polymerised and so implanted lens after implant.

It is now a further object of the invention to design a liquid composition that is reversibly photo-cross-linkable (in situ or not) to a cohesive, transparent and flexible material and product or element that at the same time or later on allows a repeatedly adjustable and reversible change of some optical characteristics of the cross-linked material,in particular by an appropriate irradiation treatment allowing to model the shape of the material and thus adjust the diopter.

It is another object of the invention to realise a conventional cross-linked material of any desired and predetermined shape and swell it in the liquid composition of the invention so as to induce a reversibly adjustable shape and thus diopter. With respect to a further object of the invention, namely its particular application to IOLs, also multi-focal IOLs, the invention aims at the realisation of an exactly adjustable IOL to the required visual acuity upon implantation and capable of repeated adjustment in vivo after implantation when needed and without invasive chirurgical treatment, even years after its implantation.

For other optical elements it permits likewise tailor made optical properties through repeated appropriate irradiation treatments on the material or element. It is thus also an object of the invention to produce transparent optical elements starting from the suitable photo-crosslinking compositions and capable of repeated adjustment of their optical properties afterwards through further suitable irradiation treatments.

### SUMMARY OF THE INVENTION

The objects of the invention are surprisingly met by an organic composition as recited in the claims. The invention thus provides in the first instance a liquid composition that can be reversibly turned to a transparent solid by light irradiation without need of varying the temperature. The liquid composition comprises polymer chains bearing some photoactive groups able to be reversibly coupled by light irradiation at a specific wavelength L1 (leading to the cross-linking of the polymer chains) and reversibly photo-cleaved when irradiated at another specific wavelength L2, the photoactive groups being capable in this way to form a polymer network playing the role of photo-reversible cross-linking nodes. The shape of such photosensitive network can thus be repeatedly and reversibly adjusted by (i) local irradiation of the composition with electromagnetic waves at the wavelength L2 (ii) by osmotic migration of the formed liquid free chains in the as-obtained partially uncross-linked network and (iii) by fixing the final new desired shape by stopping the diffusion by irradiating the whole material with a wavelength L1 that restores complete cross-linking.

The liquid composition can be irradiated in situ in a receptacle or mould. The receptacle can have at least one flexible wall, e.g. as in the capsular bag for an eye lens. The different wave lengths L1 and L2 are preferably chosen in the spectrum of the visible light, the near infrared or the near UV spectrum. The local irradiation can be a laser assisted irradiation.

With the terms "liquid composition" is meant here "liquid" at the temperature where it has to be first placed in a mould or receptacle. For application of the invention in vivo in IOL's the composition should thus be liquid (i.e. pourable or injectable) in the range of 30°C up to 40°C.

The cross-linking reaction that builds up the network is a photo-dimerisation reaction. The photo-active groups are included in the composition as chain-ends functionalities and/or pendant groups along the main chain. For cross-linking to occur, at least some multifunctional (having 3 or more photo-active groups per molecule) chains or crosslinkers have to be part of the composition. The composition can be hydrophilic or hydrophobic. The composition can also comprise a pre-existing polymeric network as a matrix swollen by the liquid photoactive composition.

### DETAILED DESCRIPTION

The modification of the optical properties is based on the use of novel polymer networks, characterised by photo-reversible cross-links. Upon laser irradiation at a specific wavelength L1, the liquid precursors of the network can be cross-linked without the help of a photo-activator. The laser irradiation of the network at a second well-defined wavelength L2 provokes the reverse reaction leading to the controlled rupture or cleavage of the cross-links.

These (photoactive) precursors can be used as an injectable liquid composition that can be cross-linked by photo-induction or can be inserted in a pre-existing network upon swelling. By using local laser irradiation, the photo-cross-linking can be induced only in a desired portion of the optical element, in particular of a lens, leading then to a difference in the *mobility* [osmotic diffusion] between the cross-linked and uncross-linked regions. This causes the diffusion of uncross-linked *liquid chains* within the element. If the lens or other optical element possesses sufficient elasticity, this migration can swell the element in the non-irradiated area which changes the shape of the lens and consequently its optical properties.

A dioptre modification of the optical element by local laser irradiation at a L2 wavelength can lead to partial rupture *(or cleavage)* of the cross-links. This is then followed by osmotic migration of the liquid molecule and further by irradiation at L1 to induce e.g. complete cross-linking resulting in solidification and fixation of the novel shape of the element.

One key aspect of the present invention lies in the choice of the chemical group performing the photo-induced cross-linking which allows the reverse reaction to occur, depending on the choice of L1 and L2. This makes the process repeatedly feasible.

The composition is formed of a polymer that preferably is liquid enough to allow the migration of the uncross-linked chains as well as a photo-induced shape adjustment. The polymers are preferably selected from polyethylene glycol oligomers or polymers, polyacrylates or polysiloxanes. They include photo-chemically active groups, e.g. a mixture of bifunctional together with multifunctional photosensitive polymers or oligomers, able to cross-link reversibly upon irradiation at selected wavelengths.

The reversible cross-linking is preferably insured by a reversible photodimerisation reaction by 2+2 cycloaddition to form a cyclobutane ring. Examples of groups allowing this reaction are coumarine, thymine, anthracene, cinnamic acid groups, cinnamates, or others resulting in cyclobutanes upon photodimerisation. This groups are covalently attached to the two chain ends of bifunctional polymers, or to the chain ends of multifunctional (star shaped) (macro)molecules and/or as pendent group along the main chain of polymers or oligomers. These photosensitive polymers can be used in mixtures so that a network is formed upon irradiation. They can also be introduced in a preformed polymer network matrix.

Illustrative examples of a suitable first polymer network matrix include also polyacrylates, poly(meth)acrylates e.g. PMMA, PHEMA and HPMA; polyvinyls and polyvinylpyrrolidone (PNVP), polyphosphazenes, and polyurethanes and copolymers thereof. This preformed network has the ability to be swollen by the photoactive liquid composition to allow liquid chains migration within the network. It is typically formed by irreversible covalent cross-linking of a polymer of similar nature as the chains of the photoactive composition. Hydrogel *networks* such as polyethylene glycols (PEG), polyurethanes or polyhydroxyethylmethacrylate based networks and hydrophobic networks such as polymethylmethacrylate or polyurethanes or polydimethylsiloxane (PDMS) based networks are preferred.

Hydrophilic *compositions* based on (i) mixture of linear and star chains of polyethylene glycols (PEG) end-capped with coumarine or (ii) mixture of linear chains of poly(hydroxyethylmethacrylate) bearing pendent coumarine and hydrophobic *compositions* based on (i) mixture of linear and star chains such as poly(methylmethacrylate) end-capped with coumarine or (ii) mixture of poly(dimethylsiloxane) (PDMS) bearing pendent coumarines are preferred.

### EXAMPLE 1.

Photoreversible hydrophilic compositions based on coumarin-functionalised polyethyleneglycol (photoactive PEGs, abreviated as COU-PEG-COU) were obtained by esterification of a coumarin acid chloride derivative with PEG diols of different molecular weights. 3-arms and 4-arms star hydroxyPEGs were similarly end-capped with coumarin. These linear and star PEGS were mixed together to form a liquid or viscous composition. The mixture was then placed in a mold and irradiated at L1>300nm during 10 min. This treatment fully converted the liquid into a transparent solid reproducing quite precisely the shape of the mould. This network is strong enough to resist manipulation and implantation if required. In general thus for optical elements these compositions according to the invention can be poured or injected into a suitable receptacle or mould.

For IOLs this composition can be formed in a mould and the preformed but still adjustable lens is then either implanted or formed in vivo. This receptacle or mould is then the emptied capsular bag of the human eye lens. Subsequently the composition of the so filled receptacle is solidified in situ in this receptacle to form a shaped body of cross-linked material.

### EXAMPLE 2

Photoactive hydrophobic compositions based on coumarin-functionalized polydimethylsiloxanes were obtained by reaction of coumarin acid chloride on linear dihydroxypropyl PDMS and linear aminopropylmethylsiloxane-co-dimethylsiloxane copolymers. These PDMS end-capped and bearing pendent coumarin groups were then mixed and cross-linked by irradiation at L1 >300 nm during 10min.. Here again, the starting liquid is rapidly converted to an elastic solid reproducing the mould shape accurately.

Then the network was locally irradiated at the center of the body with L2=254 nm until the desired shape is achieved. During this process, the body center clearly became thinner while the non- illuminated part around thickened. After a desired time (some min.), the full body was again irradiated at L1>300nm during 10min. The new body shape was thus again fixed after that irradiation).

By repeating this irradiation cycle (with L1 and L2), the adjustment can be made reversible in the two directions (cross-linking and/or cleaving), even a great number of times and even after months (expectedly years). It will be clear that the application of this process on IOLs is a great advantage since it can be done in vivo without any surgical intervention and each time that the vision capacity of the eye undesirably changes due to ageing or other causes.

The invention is not limited to the particular embodiments and examples described hereinbefore. It is understood that various changes and alterations can be conceived or applied without departing from the spirit and scope of the invention. It will be readily appreciated from the present disclosure by those skilled in the art that existing and future alternative compositions, methods or steps can perform substantially the same function or achieve substantially the same result as described or teached herein.

As an example, when multifocal IOLs are implanted in a patient, and if the patient does not get used to this multifocal IOL after a while, it must today be extracted by a surgical operation in order to replace it with monofocal IOL. When the IOL is built from the said reajustable material according to the invention the multifocality can be erased by simple irradiation at L2 = 254nm in situ in the eye without need of invasive surgery. Multifocal IOL can thus be easily converted to monofocal IOL in situ with better comfort for the patient.

## Claims

1. An organic liquid composition comprising preformed polymeric chains having some photoactive groups per chain that can reversibly couple, being capable in this way to form a transparent solid polymer network with a shape that can be repeatedly and reversibly adjusted by at least local irradiation of the network with electromagnetic waves of different wave lengths whereby the liquid composition is cross-linked at at least one wave length L1 on the one hand and is liquid again by cleaving the cross-link nodes by irradiating the material at at least one other wavelength L2 on the other hand in order to repeatedly adjust the optical properties of said composition in its photo-cross linked state.

2. Liquid composition according to claim 1 wherein the composition can be irradiated in situ in a receptacle or a mould.

3. Composition according to claim 2 wherein the receptacle can have at least one flexible wall, such as a bag.

4. Composition according to claim 1 wherein the different wave lengths L1 and L2 are chosen in the spectrum of the visible light, the near infrared or the near UV spectrum.

5. Composition according to claim 1 wherein the at least local irradiation can be a laser assisted irradiation.

6. Composition according to claim 1 wherein the cross linked state is the result of a reversible photo-dimerisation.

7. Composition according to claim 1 comprising a pre-existing polymeric network as a matrix swollen by the photoactive composition.

8. Composition according to claim 1 wherein the polymeric chains are of the type polyethylene glycol, polysiloxanes, polyurethanes, or polymethacrylates.

9. Composition according to claim 1 wherein the preformed polymeric chains have a linear or star shaped structure.

10. Composition according to claim 1 wherein the photoactive groups per chain consist of coumarine

11. A synthetic polymerised transparent and shaped optical element having a repeatedly and reversibly adjustable shape and/or optical properties by using a composition according to any of the previous claims.

12. Synthetic optical element according to claim 9 having the shape of a lens.

13. Synthetic optical element according to claim 9 having the shape of a prism.
